(19) Europäisches Patentamt
European Patent Office
Office européen des brevets

(11) **EP 1 565 563 B1**

(12) **EUROPÄISCHE PATENTSCHRIFT**

(45) Veröffentlichungstag und Bekanntmachung des Hinweises auf die Patenterteilung:
**26.09.2012   Patentblatt 2012/39**

(21) Anmeldenummer: 03780023.2

(22) Anmeldetag: **21.11.2003**

(51) Int Cl.:
***C12P 7/62*** (2006.01)

(86) Internationale Anmeldenummer:
**PCT/EP2003/013106**

(87) Internationale Veröffentlichungsnummer:
**WO 2004/048585 (10.06.2004 Gazette 2004/24)**

(54) **ENZYMATISCHE SYNTHESE VON POLYOLACRYLATEN**

ENZYMATIC SYNTHESIS OF POLYOL ACRYLATES

SYNTHESE ENZYMATIQUE D'ACRYLATES DE POLYOL

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **22.11.2002   DE 10254642**

(43) Veröffentlichungstag der Anmeldung:
**24.08.2005   Patentblatt 2005/34**

(73) Patentinhaber: **BASF SE**
**67056 Ludwigshafen (DE)**

(72) Erfinder:
• **PAULUS, Wolfgang**
**55270 Ober-Olm (DE)**
• **HAUER, Bernhard**
**67136 Fussgönheim (DE)**
• **HÄRING, Dietmar**
**69198 Schriesheim (DE)**
• **DIETSCHE, Frank**
**69198 Schriesheim (DE)**

(56) Entgegenhaltungen:
EP-A- 0 317 860      EP-A- 0 351 534
EP-A- 0 523 681      DE-A- 3 803 972
DE-A1- 19 856 948      US-A- 4 534 916
US-A- 5 240 835

• **DATABASE WPI Section Ch, Week 198610 Derwent Publications Ltd., London, GB; Class A96, AN 1986-064968 XP002278747 & JP 61 015898 A (AJINOMOTO KK), 23. Januar 1986 (1986-01-23)**
• **DATABASE WPI Section Ch, Week 198918 Derwent Publications Ltd., London, GB; Class A25, AN 1989-135527 XP002278748 & JP 01 081812 A (TOYO INK MFG CO), 28. März 1989 (1989-03-28)**
• **WARWEL SIEGFRIED ET AL: "AN EFFICIENT METHOD FOR LIPASE-CATALYSED PREPARATION OF ACRYLIC AND METHACRYLIC ACID ESTERS", BIOTECHNOLOGY TECHNIQUES, CHAPMAN & HALL LNKD- DOI:10.1007/BF00184030, vol. 10, no. 4, 1 April 1996 (1996-04-01), pages 283-286, XP008069801, ISSN: 0951-208X**

EP 1 565 563 B1

**Beschreibung**

Die Erfindung betrifft ein Verfahren zur enzymatischen Synthese von Polyolacrylaten sowie ein Verfahren zur Herstellung polymerer Polyolacrylate und Verfahren zur Herstellung von strahlungshärtbaren und/oder thermisch härtbaren Lacken, umfassend das Verfahren zur Synthese von Polyolacrylaten.

Stand der Technik:

Die Polyolacrylate sind auf verschiedenen Wegen zugänglich. Die chemische Synthese von Polyolacrylaten erfolgt durch direkte Ver- oder Umesterung von Acrylsäure oder Acrylsäureestern mit Polyolen und verläuft bei Temperaturen von über 100 °C unter Säurekatalyse. Wegen der hohen Temperaturen ist der Zusatz großer Mengen von Polymerisationsinhibitoren notwendig. Es entstehen komplexe und oft dunkle Produktgemische. Diese werden entweder durch aufwendige alkalische Auswaschungen mitsamt der überstöchiometrischen Acrylsäure aus dem Produktlösung entfernt oder verbleiben im Produkt. Das Waschverfahren ist langwierig und teuer, da sich vor allem teilveresterte Produkte nur langsam extrahieren lassen und aufgrund der höheren Hydrophilie der Produkte schlechte Ausbeuten ergeben. Die Zusammensetzung bei höheren Polyolen ist aufgrund des hohen Acrylsäureüberschusses zu den höheracrylierten Produkten verschoben. Diese sind in thermisch härtenden Systemen nicht erwünscht, da sich diese Produkte aus dem Film herauslösen, an die Oberfläche diffundieren und für die Verwendung sehr negativ, als weichmachende Komponente in allein thermisch gehärtenden Filmen klebrige Oberflächen ergeben können (siehe V1).

Alternativ sind Polyolacrylate durch ringöffnende Addition von Oxiranen mit Acrylsäure zugänglich. Diese Produkte sind in der Regel durch ein breites Nebenproduktspektrum gekennzeichnet, da sich die Ausgangsstoffe aus Umsetzungen von Alkoholen mit Epichlorhydrin ergeben, d.h. der Chlorgehalt ist durch die nicht-regioselektive Reaktion sehr hoch.

Bei der biokatalytischen Synthese beschreitet man bisher in wesentlichen zwei verschiedene Wege. Der erste Herstellungsweg verläuft über die Verwendung aktivierter Acrylsäurederivate. Insbesondere sind derartige biokatalytische Synthesen mit Vinyl(meth)acrylat (z.B. Derango et al., Biotechnol Lett. 1994, 16, 241-246); Butandiolmonooximester von (Meth)acrylsäure (Athawale und Manjrekar, J. Mol. Cat. B Enzym. 2000, 10, 551-554) oder Trifluoroethyl (meth)acrylat (Potier et al., Tetrahedron Lett. 2000, 41, 3597-3600) bekannt. Solche aktivierten Acrylsäurederivate sind aber wegen ihrer hohen Herstellkosten für eine wirtschaftliche Synthese von Polyolacrylaten nicht von Interesse.

Die Biokatalytische Herstellung von Alkololacrylaten kann auch durch enzymatische Ver- oder Umesterung von Acrylsäure oder Alkylacrylaten mit verschiedenen Alkoholen erfolgen.

So beschreibt beispielsweise die JP-A- 59220196 die Veresterung von Acrylsäure mit Diolen in wässerigem Phosphatpuffer mit Hilfe eines Enzymrohextrakts aus *Alcaligenes sp.* und ungesättigte Fettalkohole können mit Methyl- oder Ethylacrylat enzymatisch umgeestert werden (Warwel et al., Biotechnol Lett. 1996, 10, 283-286). Nurok et al. (J. Mol. Cat. B Enzym. 1999, 7, 273-282) beschreibt die Lipase-katalysierte Umesterung von 2-Ethylhexanol mit Methylacrylat. Die enzymatische Umesterung von cyclischen und offenkettigen Alkandiolen mit Ethylacrylat gelingt mit einer Lipase aus *Chromobacterium viscosum* (Hajjar et al., Biotechnol. Lett. 1990, 12, 825-830). In der US-A-5,240,835 (Genencor International Inc., 1989) wird die Umesterung von Alkylacrylaten mit Alkoholen unter Katalyse eines Biokatalysators aus *Corynebacterium oxydans* beschrieben. Beispielhaft wird dort die Reaktion von einem 96-fachen molaren Überschuß Ethylacrylat mit 2,2-Dimethyl-1,3-propandiol aufgeführt. Lediglich 21 % Ausbeute wurden nach 3 Tagen bei 30 °C erhalten. Tor et al. (Enzym. Microb. Technol. 1990, 12, 299-304) veresterten Ethylenglykol, Diethylenglykol, Triethylenglykol, Propylenglykol, 1,4-Butandiol und Glycerin mit Methyl- oder Ethyl(meth)acrylat. Die Reaktionen wurden von der Esterase aus Schweineleber (PLE) katalysiert, die mit Glutardialdehyd und Polyacrylamid- Hydrazid behandelt worden war. Diese spezielle Vorbehandlung des Enzyms war notwendig, um es gegenüber der wässerigen Substratlösung zu stabilisieren. Die Verresterung von Glycerin erfolgte bei einer Substratkonzentrat von 20 mM und die Lösung enthielt 30 Vol.-% eines 50 mM Phosphatpuffers. (vgl. auch IL 090820, 1989). In der EP-A-999 229 (Goldschmidt AG, 1999) wird die Lipase-katalysierte Umesterung von (Meth)Acrylsäure oder Alkyl(meth)acrylaten mit Polyoxyalkylenen (z.B. Polyethylenglykol) beschrieben. Geeignete Polyoxyalkylene enthalten 4-200, bevorzugt 8-120 Oxyalkyleneinheiten.

Ein Verfahren zur enzymatischen Synthese von Zuckeracrylaten ist in der älteren DE-A-101 56 352.3 beschrieben.

Die US-A-5 240 835 offenbart die Umesterung von Acrylsäureestern mit Polyolen in organischer Lösung unter Verwendung eines Biokatalysators aus *Corynebacterium glutamicum*, wie ganze Zellen, zellfreien Extrakten oder teilweise isolierten Enzymen, insbesondere Transacylasen.

Die Lipase-katalysierte Veresterung von Polyolen mit Dicarbonsäuren ist im Stand der Technik beschrieben (siehe WARWEL S. ET AL: "An efficient method for lipase-catalysed preparation of acrylic and methacrylic acid esters", BiOTECHNOLOGY TECHNIQUES, Bd. 10, Nr. 4, 1. April 1996, Seiten 283-286). Zudem ist im Stand der Technik die Lipase-katalysierte Umesterung von Acrylmethylestern bzw. Methacrylmethylestern mit ungesättigten Fettsäurealkoholen in verschiedenen organischen Lösungsmitteln beschrieben (siehe DE 198 56 948 A1).

Die biokatalytische Herstellung von Acrylaten mehrwertiger (3 und mehr Hydroxylgruppen) Alkohole, speziell aliphatischer cyclischer oder nichtcyclischer, ist jedoch bisher noch nicht beschrieben worden. Insbesondere ist aus dem Stand der Technik die enzymatische Herstellung niedrigacrylierter, aliphatischer Polyole, d.h. nicht vollständig acrylierter Polyole, nicht bekannt.

Diese Verbindungen sind für eine Verwendung in zweifach härtenden Systemen besonders interessant. Zum einen möchte man die sehr positiven mechanischen Eigenschaften strahlenhärtbarer Lacke mit der zusätzlichen Möglichkeit der thermischen Härtung infolge der unvollständigen Härtung in Schattenbereichen bei der Lackierung von dreidimensionalen Objekten kombinieren. Ziel ist eine hochkratzfeste, geruchsfreie und klebefrei Oberfläche auf unterschiedlichen Substraten. Diese sind mit derzeitigen Produkten nur schwer herstellbar, da bei der konventionellen Veresterung sehr hohe Anteile an vollständig acrylierten oder vollständig nicht-acrylierten Produkten anfallen, sind diese entweder nach der rein thermischen oder rein strahlenhärtbaren Härtung noch extrahierbar.

Kurze Beschreibung der Erfindung

Aufgabe der Erfindung war es daher, ein Verfahren zur Herstellung von Acrylaten höherwertiger aliphatischer Alkohole zu entwickeln. Die Synthese sollte insbesondere bei guter Ausbeute an niedrigacrylierten Produkten, wie z.B. Polyol-Mono- und Polyol-Di-acrylat, durchführbar sein, aber auch zu vollständig veresterten Produkten führen Insbesondere sollte auf eine wässrige Aufarbeitung/Extraktion der Produkte verzichtet werden. Obige Aufgabe konnte überraschenderweise durch gezielte Wahl der Verfahrensbedingungen, insbesondere durch Arbeiten in einem organischen Milieu, gelöst werden.

Detaillierte Beschreibung der Erfindung

Ein erster Gegenstand der Erfindung betrifft ein Verfahren zur enzymatischen Synthese von Polyolacrylaten, wobei man ein aliphatisches Polyol in Gegenwart eines unter Lipasen (E.C.3.1.1.3) ausgewählten Acrylatgruppen übertragenden Enzyms in Substanz oder in einem ein organisches Lösungsmittel umfassendes, flüssiges Reaktionsmedium mit einer Acrylsäureverbindung oder einem Alkylester davon umsetzt und man das (die)gebildete(n) Polyolacrylat(e) nach Beendigung der Reaktion gegebenenfalls aus dem Reaktionsgemisch isoliert.

Ein "aliphatisches Polyolacrylat" in Sinne der Erfindung ist ein- oder mehrfach acryliert.

Vorzugsweise erhält man bei Durchführung des erfindungsgemäßen Verfahrens ein Reaktionsprodukt welches, bezogen auf den Gesamtgehalt an acrylierten Polyolen, niedrigacrylierte Polyole in einem molaren Anteil von etwa 20 bis 100 Mol.-%, vorzugsweise 40 bis 99 Mol.-%, insbesondere 50 bis 95 Mol.-% oder 60 bis 90 Mol.-% enthält.

In einem "niedrigacryliertes Polyol" im Sinne der Erfindung ist dabei der Quotient B/A aus acrylierbaren Hydroxylgruppen vor der Reaktion (A) und verbleibenden acrylierbaren Hydroxylgruppen nach der Reaktion (B) < 1, wie z.B. 0,1 bis 0,9 oder 0,2 bis 0,66.

Das erfindungsgemäße Reaktionsprodukt stellt vorzugsweise außerdem ein Produktgemisch dar, in welchem die Summe aus vollacrylierten und vollständig nicht-acrylierten Polyolen nach der Reaktion weniger als 20 Gew.-%, insbesondere weniger als 10 Gew.-%, jeweils bezogen auf das Gesamtgewicht des Reaktionsgemisches abzüglich des Gewichts von gegebenenfalls enthaltenem Lösungsmittel bzw. niedermolekularen Zusätzen, beträgt.

Gemäß einer speziellen Ausführungsform der Erfindung kann das erfindungsgemäße Reaktionsprodukt idadurch erhalten werden, dass man der Reaktionsmischung vollständig acrylierte Verbindungen hinzufügt und die Veresterungreaktion äquilibrieren läßt.

Der erfindungsgemäß erzielte Umsatz (molarer Anteil an Polyolacrylatestern, die mindestens eine Estergruppe tragen) liegt erfindungsgemäß bei mindestens 20 Mol-%, wie z. B. 20 bis 100 Mol.%, 40 bis 99 Mol.-%, 50 bis 95 Mol.-% oder 75 bis 95 Mol.-%, jeweils bezogen auf die eingesetzten Mole Polyol.

Das flüssige organische Reaktionsmedium kann einen anfänglichen Wassergehalt von bis zu etwa 10 Vol.-% aufweisen, ist vorzugsweise aber im wesentlichen wasserfrei. Die Reaktion kann dabei in Substanz oder falls vorteilhaft auch nach Zugabe eines geeigneten organischen Lösungsmittels erfolgen.

Als organische Lösungsmittel finden vorzugsweise solche Verwendung, die ausgewählt sind unter Monoolen, wie $C_1$-$C_6$-Alkanolen, wie z.B. Methanol, Ethanol, 1- oder 2-Propanol, tert.- Butanol und tert.-Amylalkohol, Pyridin, Poly-$C_1$-$C_4$-alkylenglykoldi-$C_1$-$C_4$-alkylethern, insbesondere Polyethylenglycoldi-$C_1$-$C_4$-alkylether, wie z.B. Dimethoxyeethan, Diethylenglycoldimethylether, Polyethylenglycoldimethylether 500, $C_1$-$C_4$-Alkylencarbonaten, insbesondere Propylencarbonat, $C_1$-$C_6$-Alkylessigsäureestern, insbesondere tert.-Butylessigsäureester, MTBE, Aceton, 1,4-Dioxan, 1,3-Dioxolan, THF, Dimethoxymethan, Dimethoxyethan, Cyclohexan, Methylcyclohexan, Toluol, Hexan sowie deren ein- oder mehrphasigen Mischungen.

Im erfindungsgemäßen Verfahren werden Acrylsäureverbindung und Polyol im allgemeinen in einem molaren Verhältnis von etwa 100:1 bis 1:1, wie z.B. im Bereich von 30:1 bis 3:1 oder 10:1 bis 5:1, eingesetzt.

Die anfängliche Polyolkonzentration liegt z.B. im Bereich von etwa 0,1 bis 20 Mol/l, insbesondere 0,15 bis 10 Mol/l.

Vorzugsweise ist das Polyol ausgewählt unter geradkettigen, verzweigten oder carbocyclischen, gesättigten oder ungesättigten Kohlenwasserstoffverbindung mit wenigstens 3 Kohlestoffatomen und wenigstens 3 (veresterbaren) Hydroxylgruppen in optisch reiner Form oder als Stereoisomerengemisch. Ungesättigte Kohlenwasserstoffverbindungen können dabei 1 oder mehrere, vorzugsweise 1, 2 oder 3 C-C-Doppelbindungen aufweisen. Gemischen solchen Polyole sind ebenfalls einsetzbar.

Das Polyol ist insbesondere ein geradkettigen oder verzweigten gesättigten Kohlenwasserstoffen mit 3 bis 30 Kohlenstoffatomen und 3 bis 10 Hydroxylgruppen.

Als bevorzugte Beispiele für brauchbare Polyole sind zu nennen: Glycerin, Di-, Tri- und Polyglycerine, niedermolekulares teil- oder vollständig hydrolisiertes Polyvinylacetat, 1,2,4-Butantriol, Trimethylolmethan, Trimethylolethan, Trimethylolpropan, Trimethylolbutan, 2,2,4-Trimethyl-1,3-pentandiol, Pentaerythrit, Ditrimethylolpropan, Dipentaerythrit, Tripentaerythrit, D-, L- und Mesoerythrit, D- und L- Arabit, Adonit, Xylit, Sorbit, Mannit, Dulcit und Inositole sowie der Mischungen und Derivate davon. Unter "Derivaten" werden insbesondere $C_1$-$C_6$-Alkylether, wie z. B. Methylether; $C_1$-$C_4$-Alkylenether, wie z.B. Ethylen- oder Propylenglycoletheroder Ester von gesättigten oder ungesättigten $C_1$-$C_{20}$ Carbonsäuren verstanden. Erfindungsgemäß eingesetzte Polyole und deren Derivate enthalten insbesondere keine Polyoxyalkylengruppen mit vier oder mehr Oxyalkyleneinheiten, wie z.B. die gemäß EP-A-0 999 229 verendeten Polyoxyalkylene. Bevorzugte Polyole oder Derivate davon enthalten keine Polyoxyalkyleneinheiten.

Die erfindungsgemäß eingesetzte "Acrylsäureverbindung" ist vorzugsweise ausgewählt unter Acrylsäure, deren Anhydride, Niedrigalkyl-d.h. $C_1$-$C_6$-Alkyl-substituierter Acrylsäure, den $C_1$-$C_{20}$-Alkylestern davon oder Ethylenglykoldiacrylaten; sowie Mischungen dieser Verbindungen. Bevorzugte $C_1$-$C_6$-Alkyl-Gruppen sind insbesondere Methyl- oder Ethylgruppen. Bevorzugte $C_1$-$C_{20}$-Alkyl- Gruppen sind z.B. Methyl, Ethyl, i- oder n-Propyl, n-, i-, sec.- oder tert.-Butyl, n- oder i-Pentyl; außerdem n-Hexyl, n-Heptyl, n-Octyl, n-Nonyl, n-Decyl, n-Undecyl, n-Tridecyl, n-Tetradecyl, n-Pentadecyl und n-Hexadecyl, und n-Octadecyl, sowie die ein oder mehrfach verzweigten Analoga davon. Bevorzugt verwendet man (Meth)Acrylsäure oder (Meth)Acrylsäurederivate.

Geeignete Derivate obiger Acrylsäureverbindungen, wie z.B. Acrylsäure und (Meth)Acrylsäure, sind Ester mit gesättigten und ungesättigten, cyclischen oder offenkettigen $C_1$-$C_{10}$-Monoalkoholen, insbesondere die Methyl-, Ethyl-, Butyl- und 2-Ethylhexyl ester. davon. Die erfindungsgemäßen $C_1$-$C_{10}$-Monoalkohole umfassen bevorzugt $C_1$-$C_6$-Alkylgruppen obiger Definition oder deren längerkettigen, gegebenenfalls verzweigten, Homologen mit bis zu 10 Kohlenstoffatomen oder $C_4$-$C_6$-Cycloalkyl Gruppen, wie Cyclopropyl, Cyclopentyl oder Cyclohexyl, welche gegebenenfalls durch eine oder mehrere Alkylgruppen mit 1 bis 3 C-Atomen substituiert sein können.

Werden keine anderen Angaben gemacht so steht erfindungsgemäß $C_1$-$C_6$-Alkyl für Methyl, Ethyl, n- oder i-Propyl, n-, sec.- oder tert.-Butyl; n- oder tert.-Amyl, sowie geradkettiges oder verzweigtes Hexyl. $C_3$-$C_6$-Alkyl steht insbesondere für n- oder i-Propyl, n-, sec.- oder tert.-Butyl, n- oder tert.-Amyl, sowie geradkettiges oder verzweigtes Hexyl. $C_1$-$C_4$-Alkylen steht vorzugsweise für Methylen, Ethylen, Propylen oder 1- oder 2-Butylen.

Die erfindungsgemäß eingesetzten Enzym sind ausgewählt unter Lipasen (E.C. 3.1.1.3) in freier oder immobilisierter Form. Besonders geeignet sind Novozyme 435 (Lipase aus *Candida antartica* B) oder Lipase aus Aspergillus sp., Burholderia sp., Candida sp., Pseudomonas sp., oder Schweinepankreas. Der Enzymgehalt im Reaktionsmedium liegt insbesondere im Bereich von etwa 0,1 bis 10 Gew.-%, bezogen auf das eingesetzte Polyol. Die Enzyme können in der erfindungsgemäßen Umsetzung in reiner Form oder geträgert (immobilisiert) eingesetzt werden.

Das erfindungsgemäße Verfahren wird vorzugsweise so durchgeführt, dass die Reaktionstemperatur im Bereich von 0 bis etwa 100°C wie insbesondere im Bereich von 20 bis 80°C liegt. Die Reaktionsdauer liegt gewöhnlich im Bereich von etwa 3 bis 72 Stunden.

Der gegebenenfalls bei der Umesterung anfallende Alkohol (in der Regel ein einwertiger Alkohol, wie z.B. Methanol oder Ethanol) oder das bei der Veresterung anfallende Reaktionswasser kann, falls erforderlich, in geeigneter Weise aus dem Reaktionsgleichgewicht, kontinuierlich oder schrittweise, entfernt werden. Hierzu eignen sich vorzugsweise Molekularsiebe (Porengröße z.B. im Bereich von etwa 3-10 Angström), oder eine Abtrennung durch Destillation, durch geeignete semipermeable Membranen oder durch Pervaporation. Zur Durchmischung des Reaktionsansatzes können beliebige Verfahren eingesetzt werden. Spezielle Rührvorrichtungen sind nicht erforderlich. Das Reaktionsmedium kann ein- oder mehrphasig sein und die Reaktanden werden darin gelöst, suspendiert oder emulgiert, gegebenenfalls zusammen mit dem Molekularsieb vorgelegt. Zum Start der Reaktion kann das Medium mit dem Enzympräparat versetzt werden. Die Temperatur wird während der Reaktion auf den gewünschten Wert eingestellt.

Alternativ kann die Reaktion auch so durchgeführt werden, dass das Enzym in immobilisierter Form in einem Festbettreaktor vorgelegt und der Reaktionsansatz über das immobilisierte Enzym, gegebenenfalls im Kreislauf, gepumpt wird. Reaktionswasser und/oder - alkohol können dabei ebenfalls kontinuierlich oder schrittweise aus dem Reaktionsgemisch entfernt werden.

Das erfindungsgemäße Verfahren kann diskontinuierlich, halbkontinuierlich oder kontinuierlich in herkömmlichen Bioreaktoren durchgeführt werden. Geeignete Fahrweisen und Bioreaktoren sind dem Fachmann geläufig und z.B. beschrieben in Römpp Chemie Lexikon, 9. Auflage, Thieme Verlag, Stichwort "Bioreaktor" oder Ullmann's Encyclopedia of Industrial Chemistry, 5. Aulage, Band B4, Seiten 381 ff., worauf hiermit ausdrücklich Bezug genommen wird. Der Betrieb des Reaktors und die Verfahrensführung können vom Fachmann den jeweiligen Erfordernissen der gewünschten Veresterungsreaktion angepasst werden.

Nach Beendigung der Reaktion kann man das gewünschte Polyolacrylat aus dem Reaktionsansatz isolieren, wie z.B. chromatographisch aufreinigen, und dann zur Herstellung der gewünschter Polymere oder Copolymere einsetzen.

Ein weiterer Gegenstand der Erfindung betrifft ein Verfahren zur Herstellung von polymeren Polyolacrylaten, wobei man wenigstens ein Polyolacrylat in obiger Weise herstellt; das Polyolacrylat aus dem Reaktionsgemisch gegebenenfalls abtrennt; und, gegebenenfalls zusammen mit weiteren Comonomeren, polymerisiert.

Geeignete weitere Comonomere sind: andere erfindungsgemäß hergestellte Polyolacrylate des erfindungsgemäßen Typs oder polymerisierbare Monomere, wie (Meth)Acrylsäure, Maleinsäure, Itaconsäure, deren Alkali- oder Ammoniumsalze und deren Ester, O-Vinylester von $C_1$-$C_{25}$-Carbonsäuren, N-Vinylamide von $C_1$-$C_{25}$-Carbonsäuren, N-Vinylpyrrolidon, N-Vinylcaprolactam, N-Vinyloxazolidon, N-Vinylimidazol, quatemisiertes N-Vinylimidazol, (Meth)acrylamid, (Meth)acrylnitril, Ethylen, Propylen, Butylen, Butadien, Styrol. Beispiele für geeignete $C_1$-$C_{25}$-Carbonsäuren sind gesättigte Säuren, wie Ameisen-, Essig-, Propion- und n- und i-Buttersäure, n- und i-Valeriansäure, Capronsäure, Ö-nanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure und Melissinsäure.

Die Herstellung solcher Polymere erfolgt beispielsweise in Analogie zu den in "Ullmann's Enzyclopedia of Industrial Chemistry, Sixth Edition, 2000, Electronic Release, Stichwort: Polymerisation Process" allgemein beschriebenen Verfahren. Vorzugsweise erfolgt die (Co)polymerisation als radikalische Polymersiation in Form der Lösungs-, Suspensions-, Fällungs- oder Emulsionspolymerisation oder durch Polymerisation in Substanz, d.h. ohne Lösemittel.

Ein weiterer Gegenstand der Erfindung betrifft ein Verfahren zur Herstellung von polymeren Polyolacrylaten, wobei man wenigstens ein Polyolacrylat in obiger Weise herstellt; das unvollständig veresterte Polyolacrylat aus dem Reaktionsgemisch gegebenenfalls abtrennt; und, gegebenenfalls zusammen mit weiteren Comonomeren, polymerisiert.

Geeignete weitere Comonomere sind z.B.: andere erfindungsgemäß hergestellte Polyolacrylate des erfindungsgemäßen Typs oder polymerisierbare Monomere, wie z.B. Ethylenoxid und Propylenoxid.

Die Herstellung solcher Polymere erfolgt metallkatalysiert ohne alkalische Esterspaltung, wie sie z.B. US 6,359,101, DE 198 17 676, DE 199 13 260, US 6,429,342; US 6,077,979 und US 5,545,601 sind.

Ein weiterer Gegenstand der Erfindung betrifft die Verwendung der erfindungsgemäßen Polyolacrylate zur Herstellung von Lacken und insbesondere strahlungshärtbaren Massen, wieinsbesondere strahlungshärtbarenLacken. Dazu werden Polyolacrylate, wie z.B. Glycerinacrylate, Trimethylolpropantriacrylate oder Pentaerythritacrylate, in Form ihrer Mono-, Di- oder Polyacrylate (bzw. deren Mischungen) als Homo- oder Copolymere für strahlungshärtende Lacke z.B. in Dual Cure Systemen eingesetzt. Derartige Systeme sind z.B. in der WO-A-98/00456 beschrieben, worauf hiermit ausdrücklich Bezug genommen wird.

Neben den nach dem erfindungsgemäßen Verfahren erhältlichen Polyolacrylaten (A) kann eine erfindungsgemäße strahlungshärtbare Masse noch folgende Komponenten enthalten:

(B) mindestens eine von (A) verschiedene polymerisierbare Verbindung mit mehreren copolymerisierbaren, ethylenisch ungesättigten Gruppen,

(C) gegebenenfalls Reaktiwerdünner,

(D) gegebenenfalls Photoinitiator sowie

(E) gegebenenfalls weitere lacktypische Additive.

Als Verbindungen (B) kommen strahlungshärtbare, radikalisch polymerisierbare Verbindungen mit mehreren, d.h. mindestens zwei, copolymerisierbaren, ethylenisch ungesättigten Gruppen in Betracht.

Bevorzugt handelt es sich bei Verbindungen (B) um Vinylether- oder (Meth)acrylatverbindungen, besonders bevorzugt sind jeweils die Acrylatverbindungen, d.h. die Derivate der Acrylsäure.

Bevorzugte Vinylether- und (Meth)acrylat-Verbindungen (B) enthalten bis 20, bevorzugt bis 10 und ganz besonders bevorzugt bis 6, wie z.B. 2, 3, 4 oder 5 copolymerisierbare, ethylenisch ungesättigte Doppelbindungen.

Besonders bevorzugt sind solche Verbindungen (B) mit einem Gehalt an ethylenisch ungesättigte Doppelbindungen von 0,1 - 0,7 mol /100 g, ganz besonders bevorzugt 0,2 - 0,6 mol/100 g.

Das zahlenmittlere Molekulargewicht $M_n$ der Verbindungen (B) liegt, wenn nicht anders angegeben, bevorzugt unter 15000, besonders bevorzugt bei 300 - 12000, ganz besonders bevorzugt bei 400 bis 5000 und insbesondere bei 500 - 3000 g/mol (bestimmt durch Gelpermeationschromatographie mit Polystyrol als Standard und Tetrahydrofuran als Elutionsmittel).

Als Beispiele für Verbindungen (B) seien genannt: (Meth)acrylatverbindungen ,wie (Meth)acrylsäureester und insbesondere Acrylsäureester; sowie Vinylether von ein- oder mehrwertigen Alkoholen, insbesondere solchen, die neben den Hydroxylgruppen keine weiteren funktionellen Gruppen oder allenfalls Ethergruppen enthalten. Beispiele einwertiger Alkohole sind insbesondere Methanol, Ethanol und n- und i-Propanol. Beispiele solcher mehrwertigen Alkohole sind z.B. bifunktionelle Alkohole, wie Ethylenglykol, Propylenglykol und deren höher kondensierte Vertreter, z.B. wie Diethylenglykol, Triethylenglykol, Dipropylenglykol, Tripropylenglykol etc., 1,2-, 1,3- oder 1,4-butandiol, 1,5-pentandiol, 1,6-Hexandiol, 3-Methyl-1,5-pentandiol, Neopentylglykol, alkoxylierte phenolische Verbindungen, wie ethoxylierte bzw. propoxylierte Bisphenole, 1,2-, 1,3- oder 1,4-Cyclohexandimethanol, trifunktionelle und höherfunktionelle Alkohole, wie Glycerin, Trimethylolpropan, Butantriol, Trimethylolethan, Pentaerythrit, Ditrimethylolpropan, Dipentaerythrit, Sorbit, Mannit und die entsprechenden alkoxylierten, insbesondere ethoxylierten und/oder propoxylierten Alkohole.

Die Alkoxylierungsprodukte sind in bekannter Weise durch Umsetzung der vorstehenden Alkohole mitAlkylenoxiden, insbesondere Ethylen- oder Propylenoxyd, erhältlich. Vorzugsweise beträgt der Alkoxylierungsgrad je Hydroxylgruppe 0 bis 10, d.h. 1 mol Hydroxylgruppe kann mit bis zu 10 mol Alkylenoxiden alkoxyliert sein.

Als (Meth)acrylatverbindungen seien weiterhin Polyester(meth)acrylate genannt, wobei es sich um die (Meth)acrylsäureester oder Vinylether von Polyesterolen handelt, sowie Urethan-, Epoxid- oder Melamin(meth)acrylate.

Urethan(meth)acrylate sind z.B. erhältlich durch Umsetzung von Polyisocyanaten mit Hydroxyalkyl(meth)acrylaten und gegebenenfalls Kettenverlängerungsmitteln wie Diolen, Polyolen, Diaminen, Polyaminen oder Dithiolen oder Polythiolen.

Die Urethan(meth)acrylate haben vorzugsweise ein zahlenmittleres Molgewicht $M_n$ von 500 bis 20 000, insbesondere von 750 bis 10 000 besonders bevorzugt 750 bis 3000 g/mol (bestimmt durch Gelpermeationschromatographie mit Polystyrol als Standard).

Die Urethan(meth)acrylate haben vorzugsweise einen Gehalt von 1 bis 5, besonders bevorzugt von 2 bis 4 Mol (Meth)acrylgruppen pro 1000 g Urethan(meth)acrylat.

Epoxid(meth)acrylate sind erhältlich durch Umsetzung von Epoxiden mit (Meth)acrylsäure. Als Epoxide in Betracht kommen z.B epoxidierte Olefine oder Glycidylether, z.B. Bisphenol-A-diglycidylether oder aliphatische Glycidylether, wie Butandioldiglycidether.

Melamin(meth)acrylate sind erhältlich durch Umsetzung von Melamin mit (Meth)acrylsäure oder deren Ester.

Die Epoxid(meth)acrylate und Melamin(meth)acrylate haben vorzugsweise ein zahlenmittleres Molgewicht $M_n$ von 500 bis 20000, besonders bevorzugt von 750 bis 10000 g/mol und ganz besonders bevorzugt von 750 bis 3000 g/mol; der Gehalt an (Meth)acrylgruppen beträgt vorzugsweise 1 bis 5, besonders bevorzugt 2 bis 4 pro 1000 g Epoxid(meth)acrylat oder Melamin(meth)acrylat (bestimmt durch Gelpermeationschromatographie mit Polystyrol als Standard und Tetrahydrofuran als Elutionsmittel).

Weiterhin geeignet sind Carbonat(meth)acrylate, die im Mittel vorzugsweise 1 bis 5, insbesondere 2 bis 4, besonders bevorzugt 2 bis 3 (Meth)acrylgruppen und ganz besonders bevorzugt 2 (Meth)acrylgruppen enthalten.

Das zahlungsmittlere Molekulargewicht $M_n$ der Carbonat(meth)acrylate ist vorzugsweise kleiner 3000 g/mol, besonders bevorzugt kleiner 1500 g/mol, besonders bevorzugt kleiner 800 g/mol (bestimmt durch Gelpermeationschromatgraphie mit Polystyrol als Standard, Lösemittel Tetrahydrofuran).

Die Carbonat(meth)acrylate sind in einfacher Weise erhältlich durch Umesterung von Kohlensäureestern mit mehrwertigen, vorzugsweise zweiwertigen Alkoholen (Diolen, z.B. Hexandiol) und anschließende Veresterung der freien OH-Gruppen mit (Meth)acrylsäure oder auch Umesterung mit (Meth)acrylsäureestern, wie es z.B. in EP-A 92 269 beschrieben ist. Erhältlich sind sie auch durch Umsetzung von Phosgen, Harnstoffderivaten mit mehrwertigen, z.B. zweiwertigen Alkoholen.

Als Reaktivverdünner (Verbindungen (C)) kommen strahlungshärtbare, radikalisch oder kationisch polymerisierbare Verbindungen mit nur einer ethylenisch ungesättigten, copolymerisierbaren Gruppe in Betracht.

Genannt seien z.B. $C_1$-$C_{20}$-Alkyl(meth)acrylate, Vinylaromaten mit bis zu 20 C-Atomen, Vinylester von bis zu 20 C-Atomen enthaltenden Carbonsäuren, ethylenisch ungesättigte Nitrile, Vinylether von 1 bis 10 C-Atome enthaltenden Alkoholen, $\alpha,\beta$-ungesättigte Carbonsäuren und deren Anhydride und aliphatischen Kohlenwasserstoffen mit 2 bis 8 C-Atomen und 1 oder 2 Doppelbindungen.

Als (Meth)acrylsäurealkylester bevorzugt sind solche mit einem $C_1$-$C_{10}$-Alkylrest, wie Methylmethacrylat, Methylacrylat, n-Butylacrylat, Ethylacrylat und 2-Ethylhexylacrylat.

Insbesondere sind auch Mischungen der (Meth)acrylsäurealkylester geeignet.

Vinylester von Carbonsäuren mit 1 bis 20 C-Atomen sind z.B. Vinyllaurat, Vinylstearat, Vinylpropionat und Vinylacetat.

$\alpha,\beta$-ungesättigte Carbonsäuren und deren Anhydride können beispielsweise sein Acrylsäure, Methacrylsäure, Fumarsäure, Crotonsäure, Itaconsäure, Maleinsäure oder Maleinsäureanhydrid, bevorzugt Acrylsäure.

Als vinylaromatische Verbindungen kommen z.B. Vinyltoluol, □-Butylstyrol, 4-n-Butylstyrol, 4-n-Decylstyrol und vorzugsweise Styrol in Betracht.

Beispiele für Nitrile sind Acrylnitril und Methacrylnitril.

Geeignete Vinylether sind z.B. Vinylmethylether, Vinylisobutylether, Vinylhexylether und Vinyloctylether.

Als nicht aromatische Kohlenwasserstoffe mit 2 bis 8 C-Atomen und einer oder zwei olefinischen Doppelbindungen seien Butadien, Isopren, sowie Ethylen, Propylen und Isobutylen genannt.

Weiterhin sind N-Vinylformamid, N-Vinylpyrrolidon sowie N-Vinylcaprolactam einsetzbar.

Als Photoinitiatoren (D) können dem Fachmann bekannte Photoinitiatoren verwendet werden, z.B. solche in "Advances in Polymer Science", Volume 14, Springer Berlin 1974 oder in K. K. Dietliker, Chemistry and Technology of UV- and EB-Formulation for Coatings, Inks and Paints, Volume 3; Photoinitiators for Free Radical and Cationic Polymerization, P. K. T. Oldring (Eds), SITA Technology Ltd, London, genannten.

In Betracht kommen z.B. Mono- oder Bisacylphosphinoxide Irgacure 819 (Bis(2,4,6-Trimethylbenzoyl)phenylphosphinoxid), wie sie z.B. in EP-A 7 508, EP-A 57 474, DE-A 196 18 720, EP-A 495 751 oder EP-A 615 980 beschrieben sind, beispielsweise 2,4,6-Trimethylbenzoyldiphenylphosphinoxid (Lucirin® TPO), Ethyl-2,4,6-trimethylbenzoylphenylphosphinat, Benzophenone, Hydroxyacetophenone, Phenylglyoxylsäure und ihre Derivate oder Gemische dieser Photoinitiatoren. Als Beispiele seien genannt Benzophenon, Acetophenon, Acetonaphthochinon, Methylethylketon, Valerophenon, Hexanophenon, α-Phenylbutyrophenon, p-Morpholinopropiophenon, Dibenzosuberon, 4-Morpholinobenzophenon, 4-Morpholinodeoxybenzoin, p-Diacetylbenzol, 4-Aminobenzophenon, 4'-Methoxyacetophenon, ß-Methylanthrachinon, tert-Butylanthrachinon, Anthrachinoncarbonysäureester, Benzaldehyd, α-Tetralon, 9-Acetylphenanthren, 2-Acetylphenanthren, 10-Thioxanthenon, 3-Acetylphenanthren, 3-Acetylindol, 9-Fluorenon, 1-Indanon, 1,3,4-Triacetylbenzol, Thioxanthen-9-on, Xanthen-9-on, 2,4-Dimethylthioxanthon, 2,4-Diethylthioxanthon, 2,4-Di-iso-propylthioxanthon, 2,4-Dichlorthioxanthon, Benzoin, Benzoin-iso-butylether, Chloroxanthenon, Benzointetrahydropyranylether, Benzoin-methylether, Benzoin-ethylether, Benzoin-butylether, Benzoin-iso-propylether, 7-H-Benzoin-methylether, Benz[de]anthracen-7-on, 1-Naphthaldehyd, 4,4'-Bis(dimethylamino)benzophenon, 4-Phenylbenzophenon, 4-Chlorbenzophenon, Michlers Keton, 1-Acetonaphthon, 2-Acetonaphthon, 1-Benzoylcyclohexan-1-ol, 2-Hydroxy-2,2-dimethylacetophenon, 2,2-Dimethoxy-2-phenylacetophenon, 2,2-Diethoxy-2-phenylacetophenon, 1,1-Dichloracetophenon, 1-Hydroxyacetophenon, Acetophenondimethylketal, o-Methoxybenzophenon, Triphenylphosphin, Tri-o-Tolylphosphin, Benz[a]anthracen-7,12-dion, 2,2-Diethoxyacetophenon, Benzilketale, wie Benzildimethylketal, 2-Methyl-1-[4-(methylthio)phenyl]-2-morpholinopropan-1-on, Anthrachinone wie 2-Methylanthrachinon, 2-Ethylanthrachinon, 2-tert-Butylanthrachinon, 1-Chloranthrachinon, 2-Amylanthrachinon und 2,3-Butandion.

Geeignet sind auch nicht- oder wenig vergilbende Photoinitiatoren vom Phenylglyoxalsäureestertyp, wie in DE-A 198 26 712, DE-A 199 13 353 oder WO 98/33761 beschrieben.

Unter den genannten Photoinitiatoren sind Phosphinoxide, α-Hydroxyketone und Benzophenone bevorzugt.

Insbesondere können auch Gemische verschiedener Photoinitiatoren verwendet werden.

Die Photoinitiatoren können allein oder in Kombination mit einem Photopolymerisationspromotor, z.B. vom Benzoesäure-, Amin- oder ähnlichem Typ verwendet werden.

Als weitere lacktypische Additive (E) können beispielsweise Antioxidantien, Oxidationsinhibitoren, Stabilisatoren, Aktivatoren (Beschleuniger), Füllmittel, Pigmente, Farbstoffe, Entgasungsmittel, Glanzmittel, antistatische Agentien, Flammschutzmittel, Verdicker, thixotrope Agentien, Verlaufshilfsmittel, Bindemittel, Antischaummittel, Duftstoffe, oberflächenaktive Agentien, Viskositätsmodifikatoren, Weichmacher, Plastifizierer, klebrigmachende Harze (Tackifier), Chelatbildner oder Verträglichkeitsmittel (compatibilizer) verwendet werden.

Als Beschleuniger für die thermische Nachhärtung kann z.B. Zinnoctoat, Zinkoctoat, Dibutylzinnlaureat oder Diaza[2.2.2]bicyclooctan verwendet werden.

Weiterhin können ein oder mehrere photochemisch und/oderthermisch aktivierbare Initiatoren zugesetzt werden, z.B. Katiumperoxodisulfat, Dibenzoylperoxid, Cyclohexanonperoxid, Di-tert.-Butylperoxid, Azobis-iso-butyronitril, Cyclohexylsulfonylacetylperoxid, Di-isopropylpercarbonat, tert-Butylperoktoat oder Benzpinakol, sowie beispielsweise solche thermisch aktivierbare Initiatoren, die eine Halbwertszeit bei 80°C von mehr als 100 Stunden aufweisen, wie Di-t-Butylperoxid, Cumolhydroperoxid, Dicumylperoxid, t-Butylperbenzoat, silylierte Pinakole, die z. B. unter dem Handelsnamen ADDID 600 der Firma Wacker kommerziell erhältlich sind oder Hydroxylgruppen-haltige Amin-N-Oxide, wie 2,2,6,6-Tetramethylpiperidin-N-oxyl, 4-Hydroxy-2,2,6,6-Tetramethylpiperidin-N-oxyl etc. Weitere Beispiele geeigneter Initiatoren sind in "Polymer Handbook", 2. Aufl., Wiley & Sons, New York beschrieben.

Als Verdicker kommen neben radikalisch (co)polymerisierten (Co)Polymerisaten, übliche organische und anorganische Verdicker wie Hydroxymethylcellulose oder Bentonite in Betracht.

Als Chelatbildner können z.B. Ethylendiaminessigsäure und deren Salze sowie ß-Diketone verwendet werden.

Geeignete Füllstoffe umfassen Silikate, z. B. durch Hydrolyse von Siliciumtetrachlorid erhältliche Silikate wie Aerosil® der Fa. Degussa, Kieselerde, Talkum, Aluminiumsilikate, Magnesiumsilikate, Calciumcarbonate etc.

Geeignete Stabilisatoren umfassen typische UV-Absorber wie Oxanilide, Triazine und Benzotriazol (letztere erhältlich als Tinuvin® -Marken der Ciba-Spezialitätenchemie) und Benzophenone. Diese können allein oder zusammen mit geeigneten Radikalfängern, beispielsweise sterisch gehinderten Aminen wie 2,2,6,6-Tetramethylpiperidin, 2,6-Di-tert.-butylpiperidin oder deren Derivaten, z. B. Bis-(2,2,6,6-tetra-methyl-4-piperidyl)sebacinat, eingesetzt werden. Stabilisatoren werden üblicherweise in Mengen von 0,1 bis 5,0 Gew.-%, bezogen auf die in der Zubereitung enthaltenen festen Komponenten, eingesetzt.

Weiterhin geeignete Stabilisatoren sind beispielsweise N-Oxyle, wie z.B. 4-Hydroxy-2,2,6,6-tetramethyl-piperidin-N-oxyl, 4-Oxo-2,2,6,6-tetramethyl-piperidin-N-oxyl, 4-Acetoxy-2,2,6,6-tetramethyl-piperidin-N-oxyl, 2,2,6,6-tetramethyl-piperidin-N-oxyl, 4,4',4"-Tris(2,2,6,6-tetramethyl-piperidin-N-oxyl)-phosphit oder 3-Oxo-2,2,5,5-tetramethylpyrrolidin-N-oxyl, Phenole und Naphthole, wie z.B. p-Aminophenol, p-Nitrosophenol, 2-*tert.*-Butylphenol, 4-*tert.*-Butylphenol, 2,4-di-*tert.*-Butylphenol, 2-Methyl-4-*tert.*-Butylphenol, 4-Methyl-2,6-*tert.*-Butylphenol (2,6-*tert.*-Butyl-p-Kresol) oder 4-*tert.*-Butyl-2,6-dimethylphenol, Chinone, wie z.B. Hydrochinon oder Hydrochinonmonomethylether, aromatische Amine, wie z.B. N,N-Diphenylamin, N-Nitroso-diphenylamin, Phenylendiamine, wie z.B. N,N'-Dialkyl-para-phenylendiamin, wobei die Alkylreste gleich oder verschieden sein können und jeweils unabhängig voneinander aus 1 bis 4 Kohlenstoffatome bestehen und geradkettig oder verzweigt sein können, Hydroxylamine, wie z.B. N,N-Diethylhydroxylamin, Harnstoffderivate, wie z.B. Harnstoff oder Thioharnstoff, phosphorhaltige Verbindungen, wie z.B. Triphenylphosphin, Triphenylphosphit oder Triethylphosphit oder schwefelhaltige Verbindungen, wie z.B. Diphenylsulfid oder Phenothiazin.

Typische Zusammensetzungen für strahlungshärtbare Massen sind beispielsweise

(A) 20 -100 Gew%, bevorzugt 40 - 90, besonders bevorzugt 50 - 90 und insbesondere 60 - 80 Gew%,
(B) 0 - 60 Gew%, bevorzugt 5 - 50, besonders bevorzugt 10 - 40 und insbesondere 10 - 30 Gew%,
(C) 0 - 50 Gew%, bevorzugt 5 - 40, besonders bevorzugt 6 - 30 und insbesondere 10 - 30 Gew%,
(D) 0 - 20 Gew%, bevorzugt 0,5 -15, besonders bevorzugt 1-10 und insbesondere 2 - 5 Gew% sowie
(E) 0 - 50 Gew%, bevorzugt 2 - 40, besonders bevorzugt 3 - 30 und insbesondere 5 - 20 Gew%,

mit der Maßgabe, daß (A), (B), (C), (D) und (E) zusammen 100 Gew.-% ergeben.

Die Beschichtung von Substraten mit erfindungsgemäßen Beschichtungsmassen erfolgt nach üblichen, dem Fachmann bekannten Verfahren, wobei man wenigstens eine Beschichtungsmasse auf das zu beschichtende Substrat in der gewünschten Stärke aufbringt und die gegebenenfalls enthaltenen flüchtigen Bestandteile der Bechichtungsmasse, gegebenenfalls unter Erhitzen, entfernt. Dieser Vorgang kann gewünschtenfalls ein- oder mehrfach wiederholt werden. Das Aufbringen auf das Substrat kann in bekannter Weise, z. B. durch Spritzen, Spachteln, Rakeln, Bürsten, Rollen, Walzen, Gießen, Laminieren, Hinterspritzen oder Coextrudieren erfolgen. Die Beschichtungsstärke liegt in der Regel in einem Bereich von etwa 3 bis 1000 g/m$^2$ und vorzugsweise 10 bis 200 g/m$^2$.

Die erfindungsgemäß hergestellten Polymere sind auch in Verfahren zum Beschichten von Substraten einsetzbar, bei dem man die Beschichtungsmasse auf das Substrat aufbringt und gegebenenfalls trocknet, mit Elektronenstrahlen oder UV Belichtung unter sauerstoffhaltiger Atmosphäre oder bevorzugt unter Inertgas härtet, gegebenenfalls bei Temperaturen bis zur Höhe der Trocknungstemperatur und anschließend bei Temperaturen bis zu 160°C, bevorzugt zwischen 60 und 160 °C, thermisch behandelt.

Das Verfahren zum Beschichten von Substraten kann auch so durchgeführt werden, dass nach dem Aufbringen der Beschichtungsmasse zunächst bei Temperaturen bis zu 160°C, bevorzugt zwischen 60 und 160 °C, thermisch behandelt und anschließend mit Elektronenstrahlen oder UV Belichtung unter Sauerstoff oder bevorzugt unter Inertgas gehärtet wird.

Die Härtung der auf dem Substrat gebildeten Filme kann gewünschtenfalls ausschließlich thermisch erfolgen. Im allgemeinen härtet man die Beschichtungen jedoch sowohl durch Bestrahlung mit energiereicher Strahlung als auch thermisch.

Die Härtung kann auch zusätzlich oder anstelle der thermischen Härtung durch NIR-Strahlung erfolgen, wobei als NIR-Strahlung hier elektromagnetische Strahlung im Wellenlängenbereich von 760 nm bis 2,5 ☐m, bevorzugt von 900 bis 1500 nm bezeichnet ist.

Gegebenenfalls kann, wenn mehrere Schichten des Beschichtungsmittels übereinander aufgetragen werden, nach jedem Beschichtungsvorgang eine thermische, NIR und/oder Strahlungshärtung erfolgen.

Als Strahlungsquellen für die Strahlungshärtung geeignet sind z.B. Quecksilber-Niederdruckstrahler, -Mitteldruckstrahler mit Hochdruckstrahler sowie Leuchtstoffröhren, Impulsstrahler, Metallhalogenidstrahler, Elektronenblitzeinrichtungen, wodurch eine Strahlungshärtung ohne Photoinitiator möglich ist, oder Excimerstrahler. Die Strahlungshärtung erfolgt durch Einwirkung energiereicher Strahlung, also UV-Strahlung oder Tageslicht, vorzugsweise Licht im Wellenlängenbereich von λ=200 bis 700 nm strahlt, besonders bevorzugt von λ=200 bis 500 nm und ganz besonders bevorzugt λ=250 bis 400 nm, oder durch Bestrahlung mit energiereichen Elektronen (Elektronenstrahlung; 150 bis 300 keV). Als Strahlungsquellen dienen beispielsweise Hochdruckquecksilberdampflampen, Laser, gepulste Lampen (Blitzlicht), Halogenlampen oder Excimerstrahler. Die üblicherweise zur Vernetzung ausreichende Strahlungsdosis bei UV-Härtung liegt im Bereich von 80 bis 3000 mJ/cm$^2$.

Selbstverständlich sind auch mehrere Strahlungsquellen für die Härtung einsetzbar, z.B. zwei bis vier. Diese können auch in jeweils unterschiedlichen Wellenlängebereichen strahlen.

Die Bestrahlung kann gegebenenfalls auch unter Ausschluss von Sauerstoff, z. B. unter Inertgas-Atmosphäre, durchgeführt werden. Als Inertgase eignen sich vorzugsweise Stickstoff, Edelgase, Kohlendioxid, oder Verbrennungsgase. Des weiteren kann die Bestrahlung erfolgen, indem die Beschichtungsmasse mit transparenten Medien abgedeckt wird. Transparente Medien sind z. B. Kunststofffolien, Glas oder Flüssigkeiten, z. B. Wasser. Besonders bevorzugt ist eine Bestrahlung in der Weise, wie sie in der DE-A1 199 57 900 beschrieben ist.

Die erfindungsgemäß hergestellten Polymere sind auch auswendbar in einem Verfahren zur Beschichtung von Substraten, wobei man

i) ein Substrat mit einer Beschichtungsmasse, wie zuvor beschrieben, beschichtet,

ii) flüchtige Bestandteile der Beschichtungsmasse zur Filmbildung unter Bedingungen entfernt, bei denen der Photoinitiator (C) im wesentlichen noch keine freien Radikale ausbildet,

iii) gegebenenfalls den in Schritt ii) gebildeten Film mit energiereicher Strahlung bestrahlt, wobei der Film vorgehärtet wird, und anschließend gegebenenfalls den mit dem vorgehärteten Film beschichteten Gegenstand mechanisch bearbeitet oder die Oberfläche des vorgehärteten Films mit einem anderen Substrat in Kontakt bringt, und

iv) dem Film thermisch oder mit NIR-Strahlung endhärtet.

Dabei können die Schritte iv) und iii) auch in umgekehrter Reihenfolge durchgeführt, d. h. der Film kann zuerst thermisch oder per NIR-Strahlung und dann mit energiereicher Strahlung gehärtet werden.

Die Erfindung wird nun anhand folgender Beispiele näher erläutert.

Allgemeine Angaben:

A) Gaschromatographie:

Die Reaktionsprodukte von Glycerin und Trimethylolpropan mit den Acrylaten wurden gaschromatographisch auf einer Kapilarsäule CP-Sil 19 (14% Cyanopropyl-phenyl, 86% Dimethylpolysiloxane) der Firma Varian aufgetrennt. Zur GC-Analyse der Reaktionsprodukte von Sorbit und Erythrit mit Acrylaten wurden 50 $\mu$l Reaktionslösung mit 950 $\mu$l Sylon HTP (Firma Supelco) für 10 min bei 20 °C behandelt und anschließend auf einer Kappilarsäure CP-Sil 5 (100% Dimethylpolysiloxane, Firma Varian) analysiert.

B) Bestimmung des "Gesamtextrahierbaren Anteils":

Der gesamtextrahierbare Anteil thermisch gehärteter Lacke wird durch Aceton-Extraktion thermisch gehärteter Lack-Tabletten bestimmt

a) Herstellung der Lacktabletten und Prüfung:

Die zu prüfenden Lacke werden frisch angesetzt (ohne Fotoinitiator) eingewogen (5 g). Die Lack-Tabletten werden 24 h bei 60°C im Trockenschrank gehärtet. Nach der Härtung werden die Filme halbiert. Jede Hälfte wird eingewogen (Analysenwaage, je ein Becher für die Extraktion und ein Becher ohne Aceton zum Vergleich). In den einen Becher (Ac) werden 100 g Aceton gefüllt. Beide Becher werden mit Deckeln verschlossen und 24 h bei 23°C / 55% relative Luftfeuchte gelagert.

Nach der Lagerung wird das Aceton aus den Ac-Bechern geschüttet (durch ein Nylon-Sieb, um evtl. Tabletten-Bruchstücke zurückzuhalten). Alle Becher werden ohne Deckel 2h bei 80°C getrocknet und nach dem Abkühlen zurückgewogen.

b) Berechnung:

$$\frac{m_0 Lu - m_1 Lu}{mT_0 Lu} * 100 = \Delta Lu \text{ (\% Verlust luftgelagerte Tablette)}$$

$$\underline{\frac{m_0Ac - m_1Ac}{mT_0Ac}} * 100 = \Delta Ac \text{ (\% Verlust acetongelagerte Tablette)}$$

$\Delta Ac - \Delta Lu$ = % extrahierbarer Anteil
$mT_0Lu$ Masse Tablette Lu vor Lagerung in Luft
$m_0Lu$ Masse Becher + Tablette Lu vor Lagerung in Luft
$m_1Lu$ Masse Becher + Tablette Lu nach Lagerung in Luft
$mT_0Ac$ Masse Tablette Ac vor Lagerung in Aceton
$m_0Ac$ Masse Becher + Tablette Lu vor Lagerung in Aceton
$m_1Ac$ Masse Becher + Tablette Lu nach Lagerung in Aceton

c) Blindprobe

Die bei jeder Bestimmung mitgeprüfte Blindprobe (½ Tablette 24 h an Luft) dient der Erfassung eventueller Materialverluste während der Trocknung. Erfahrungsgemäß verlieren alle Blindproben bei der Trocknung 0,2% - 0,5%. Dieser Verlust wird vom Verlust der extrahierten Probe abgezogen.

Beispiel 1: Umsetzung von TMP mit Methylacrylat in MTBE

Eine Mischung aus 0,1 mol (13,4 g) Trimethylolpropan (TMP), 1,0 mol (86,1 g) Methylacrylat, 200 ml MTBE, 20 g 5 Å Molsieb und 2,0 g Novozym 435 (Lipase aus *Candida antartica* B) wurde 24 h unter Rückfluß gerührt. Das Enzym wurde abfiltriert, MTBE im Vakuum. abrotiert und 22 g Rohprodukt (klare, gelbliche Flüssigkeit) erhalten.
Eine Probe wurde entnommen, silyliert und mittels GC analysiert. Laut GC-Analyse setzte sich das Produkt wie folgt zusammen: 16 % TMP, 60 % TMP-monoacrylat, 21 % TMP-diacrylat, <1 % TMP-triacrylat.

Beispiel 2: Umsetzung von Glycerin mit Methylacrylat in MTBE, ohne Molsieb

Eine Mischung aus 125 mmol (11,5 g) Glycerin, 1,25 mol (107,6 g) Methylacrylat, 250 ml Aceton und 2,5 g Novozym 435 (Lipase aus *Candida antartica* B) wurde 2 Tage bei 40 °C geschüttelt. Das Enzym wurde abfiltriert (kann wiederverwendet werden) und Aceton im Vakuum abrotiert. Man erhielt 27 g Rohprodukt (klare, gelbliche Flüssigkeit).
Eine Probe wurde entnommen, silyliert und mittels GC analysiert. Laut GC-Analyse setzte sich das Produkt wie folgt zusammen: 6 % Glycerin, 54 % Glycerin-monoacrylat, 37 % Glycerin-diacrylat, <1 % Glycerin-triacrylat.
Gesamtextrahierbarer Anteil nach thermischer bzw. UV-Härtung: < 5 Gew.-%

Beispiel 3: Umsetzung von TMP mit Methylacrylat

a) Eine Mischung aus 0,5 mol (67 g) TMP, 5 mol (430,5 g) Methylacrylat, 100 g Molsieb (5 Å) und 10 g Novozym 435 (Lipase aus *Candida antartica* B) wurde 72 Stunden bei 60 °C gerührt. Das Enzym wurde abfiltriert und das Filtrat von den niederflüchtigen Bestandteilen destillativ abgetrennt. Es ergaben sich 142 g TMPTA (klare, farblose Flüssigkeit).

Eine Probe wurde entnommen und silyliert. Laut GC-Analyse war TMP zu >99 % umgesetzt worden,d.h. es entstand fast vollständig Triacrylat.
Gesamtextrahierbarer Anteil nach UV-Härtung: < 5 Gew.-%

b) Eine Mischung aus 0,5 mol (67 g) TMP, 5 mol (430,5 g) Methylacrylat, 100 g Molsieb (5 Å) und 10 g Novozym 435 (Lipase aus *Candida antartica* B) wurde 24 h bei 40 °C gerührt. Das Enzym wurde abfiltriert und das Filtrat von den niederflüchtigen Bestandteilen destillativ abgetrennt. Es ergaben sich 104 g Produkt (klare, farblose Flüssigkeit).

Eine Probe wurde entnommen und silyliert. Laut GC-Analyse setzte sich das Produkt wie folgt zusammen: 2 % TMP, 22 % TMP-monoacrylat, 72 % TMP-diacrylat, <3 % TMP-triacrylat.
Gesamtextrahierbarer Anteil nach thermischer bzw. UV- Härtung: < 5 Gew.-%

Beispiel 4: Umsetzung von TMP mit Acrylsäure (Vergleichsbespiel 1)

Eine Mischung aus 0,5 mol (67 g) TMP, 0,5 Gew.-% $H_2SO_4$, 1,8 mol (99 g) Acrylsäure wurde in Cyclohexan gelöst und anfallendes Reaktionswasser bis zu einem Umsatz von 50% bzw. 66% ausgekreist. Der Ansatz wurde jeweils bis zur Säurezahl 40 destillativ aufgereinigt. Es ergaben sich 108 g bzw. 120 g Produkt (klare, gelbliche Flüssigkeiten).

Eine Probe wurde entnommen und silyliert. Laut GC-Analyse setzte sich das Produkt wie folgt zusammen.

Umsatz [50 %]: 15% TMP, 45% TMP-monoacrylat, 23% TMP-diacrylat, 17% TMP-triacrylat.

Gesamtextrahierbarer Anteil nach thermischer Härtung: 33 Gew.-% (Butylacetat, Raumtemp.)

Gesamtextrahierbarer Anteil nach UV-Härtung: 47 Gew.-% (Butylacetat, Raumtemp.)

Umsatz [67 %]: 2% TMP, 15% TMP-monoacrylat, 25% TMP-diacrylat, 59% TMP-triacrylat.

Gesamtextrahierbarer Anteil nach thermischer Härtung: 64 Gew.-% (Butylacetat, Raumtemp.)

Gesamtextrahierbarer Anteil nach UV-Härtung: 27 Gew.-% (Butylacetat, Raumtemp.)

Beispiel 5: Umsetzung von Glycerin mit Ethylacrylat in tert.-Butanol

Eine Mischung aus 5 mmol (0,46 g) Glycerin, 50 mmol (5,0 g) Ethylacrylat, 10 ml tert-Butanol, 1 g Molsieb (5 Å) und 0,1 g Novozym 435 (Lipase aus *Candida antartica* B) wurde 3 Tage bei 20 °C geschüttelt.

Eine Probe wurde entnommen, silyliert und mittels GC analysiert.. Laut GC-Analyse setzte sich das Produkt wie folgt zusammen: 5 Gew.-% Glycerin, 42 Gew.-% Glycerinmonoacrylat, 53Gew. % Glycerindiacrylat und <1 Gew. % Glycerintriacrylat.

Beispiel 6: Umsetzung von Glycerin mit Methylacrylat

Eine Mischung aus 125 mmol (11,5 g) Glycerin, 1,25 mol (107,6 g) Methylacrylat, 250 ml Aceton und 2,5 g Novozym 435 (Lipase aus *Candida antartica* B) wurde 2 Tage bei 40 °C geschüttelt. Das Enzym wurde abfiltriert (und kann wiederverwendet werden). Aceton wurde im Vakuum abrotiert. Es wurden 19,4 g Rohprodukt (klare, gelbliche Flüssigkeit) erhalten.

Eine Probe wurde entnommen, silyliert und mittels GC analysiert. Laut GC-Analyse setzte sich das Produkt wie folgt zusammen: 15 Gew.-% Glycerin, 37 Gew. % Glycerinmonoacrylat, 46 Gew. % Glycerindiacrylat und <1 Gew. % Glycerintriacrylat.

Beispiel 7: Umsetzung von Glycerin und Methylacrylat in Aceton

Eine Mischung aus 0,5 mol (46,3 g) Glycerin, 5 mol (430,5 g) Methylacrylat, 500 ml Aceton, 100 g Molsieb (5 Å) und 10,0 g Novozym 435 (Lipase aus *Candida antartica* B) wurde 72 Stunden bei 20 °C gerührt. Das Enzym wurde abfiltriert (und kann wiederverwendet werden) und das Filtrat im Vakuum eingeengt. Es wurden80,9 g Rohprodukt (klare, farblose Flüssigkeit) erhalten.

Eine Probe wurde entnommen und silyliert. Laut GC-Analyse setzte sich das Produkt wie folgt zusammen: 8 Gew.-% Glycerin, 48 Gew.-% Glycerinmonoacrylat, 41 Gew. % Glycerindiacrylat und 3 Gew. % Glycerintriacrylat.

Beispiel 8: Umsetzung von Glycerin und Methylmethacrylat ohne Lösungsmittel, ohne Molsieb

Eine Mischung aus 5 mmol (0,46 g) Glycerin, 50 mmol (5,0 g) Methylmethacrylat und 0,1 g Novozym 435 (Lipase aus *Candida antartica* B) wurde 24 Stunden bei 20 °C geschüttelt.

Eine Probe wurde entnommen und silyliert. Laut GC-Analyse setzte sich das Produkt wie folgt zusammen: 15 Gew.-% Glycerin, 55 Gew.-% Glycerinmonoacrylat, 30 Gew. % Glycerindiacrylat und <1 Gew. % Glycerintriacrylat.

Beispiel 9: Umsetzung von Erythrit und Methylacrylat in tert-Butanol

50 mmol Erythrit (6,1 g), 500 mmol Methylacrylat, 300 ml tert-Butanol und 1,0 g immobilisierte Lipase aus Candida antartica (Novozym 435) wurden 72 Stunden bei 40 °C gerührt. Das Enzym wurde abfiltriert und der Überschuss Methylacrylat und das Solvens am Rotationsverdampfer im Vakuum bei 40 °C entfernt.

Man erhielt 14,1 g Wertprodukt, das laut GC-Analyse 21 Gew.-% Erythrit, 49 Gew.-% % Erythritmonoacrylat, 29 Gew.-% % Erythritdiacrylat und < 0,2 Gew.-% % Erythritdiacrylat enthielt.

Beispiel 10: Umsetzung von Sorbit mit Methylacrylat in tert-Butanol

In einem Vierhals-Rundkolben mit aufgesetztem Rückflusskühler wurden 63,8 g Sorbit (0,35 mol), 301,3 g Methylacrylat (3,5 mol), 2100 ml tert Butanol und 7,0 g lyophilisierte Lipase aus *Burholderia sp.* bei 40 °C 72 Stunden gerührt. Anschließend filtrierte man mit einer Filternutsche (D3 mit Kieselgelschicht) die Lipase und nicht gelöstes Sorbit ab und entfernte überschüssiges Methylacrylat und Lösungsmittel am Rotationsverdampfer bei 40 °C im Vakuum. Man erhielt 83,3 g Produkt.

Die GC-Analytik ergab einen Gehalt von 45 Gew.-% Sorbitmonoacrylat, 42 Gew.-% Sorbitdiacrylat, 3 Gew.-% Sorbittriacrylat und 10 Gew.-% Sorbit.

Beispiel 11: Herstellung einer gehärteten Lackschicht

a) Thermische Härtung:

Eine Mischung aus 16 Gew.-% eines Reaktionsprodukts aus Beispiel 3b bzw. 2, 50 Gew.-% Basonat HI 100, 34 Gew.-% eines Polyols sowie eine Mischung aus 3,5 Gew.-% Irgacure® 184 (Ciba Speciality Chemicals) und 0,5 Gew.-% Lucirin TPO® (BASF AG) wurden unter Zusatz von 1 Gew.-% DBTL in Butyl-Acetat gelöst und für 16 h bei 60°C thermisch gehärtet. Es entstand ein farbloser, nach 30 min klebfreier Film. Dieser wurde nach 16h abgekühlt und bei RT für 24h mit Aceton extrahiert und anschließend getrocknet.

b) UV-Härtung:

Die Beschichtungsmasse wurde fünfmal unter einer undotierten Quecksilberhochdrucklampe (Leistung 120 W/cm) mit einem Lampenabstand zum Substrat von 12 cm und einer Bandgeschwindigkeit von 5 m/min belichtet. Die Schichtdicke nach der Belichtung betrug ca. 50 $\mu$m.

Die Pendeldämpfung wurde nach DIN 53157 mit 118 bzw. 110 bestimmt und ist ein Maß für die Härte der Beschichtung. Die Angabe erfolgt in Pendelschlägen. Hohe Werte bedeuten dabei hohe Härte. Die Erichsentiefung wurde nach DIN 53156 mit 4,6 bzw. 7,0 bestimmt und ist ein Maß für die Flexibilität und Elastizität. Die Angabe erfolgt in Millimeter (mm). Hohe Werte bedeuten hohe Flexibilität. Die Haftung mit Gitterschnitt wurde gemäß der DIN 53151 ermittelt und in Noten angegeben. Kleine Werte bedeuten hohe Haftung. Es ergab sich jeweils eine 0/5 Beurteilung.

Für Vergleichsbeispiel 1 [50%] ergeben sich folgende Werte:
Pendeldämpfung: 32; Erichsentiefung: 8,9; Haftung: 1/5.

Es zeigt sich somit, dass unter Verwendung der erfindungsgemäßen Polyolacrylate Polymerbeschichtungen mit deutlich verbessertem Eigenschaftsprofil herstellbar sind.

**Patentansprüche**

1. Verfahren zur enzymatischen Synthese von Polyolacrylaten, wobei man ein aliphatisches Polyol in Gegenwart eines unter Lipasen (E.C.3.1.1.3) ausgewählten Acrylatgruppen übertragenden Enzyms in Substanz oder in einem ein organisches Lösungsmittel umfassendes, flüssiges Reaktionsmedium mit einer Acrylsäureverbindung oder einem Alkylester davon umsetzt, und man das (die) gebildete(n) Polyolacrylat(e) nach Beendigung der Reaktion gegebenenfalls aus dem Reaktionsgemisch isoliert.

2. Verfahren nach Anspruch 1, wobei eine Lipase aus *Candida sp.*, insbesondere *Candida antarctica* B, oder *Burkholderia sp.* verwendet wird.

3. Verfahren nach Anspruch 1 oder 2, wobei das flüssige Reaktionsmedium einen anfänglichen Wassergehalt von weniger als etwa 10 Vol.-% aufweist.

4. Verfahren nach einem der vorhergehenden Ansprüche, wobei Acrylsäureverbindung und Polyol in einem molaren Verhältnis von etwa 100:1 bis 1:1 eingesetzt werden.

5. Verfahren nach einem der vorhergehenden Ansprüche, wobei die Acrylsäureverbindung ausgewählt ist unter Acrylsäure, $C_1$-$C_8$-Alkyl-substituierter Acrylsäure, und den Alkylestern dieser Verbindungen, sowie Mischungen davon.

6. Verfahren nach einem der vorhergehenden Ansprüche, wobei das Polyol ausgewählt ist unter geradkettigen oder verzweigten oder carbocyclischen, gesättigten oder ungesättigten Kohlenwasserstoffverbindung mit wenigstens 3 Kohlenstoffatomen und wenigstens 3 (veresterbaren) Hydroxylgruppen in optisch reiner Form oder als Stereoisomerengemisch oder Mischungen unterschiedlicher Polyole.

7. Verfahren nach Anspruch 6, wobei das Polyol ausgewählt ist unter geradkettigen, verzweigten oder cyclischen gesättigten Kohlenwasserstoffen mit 3 bis 30 Kohlenstoffatomen und 3 bis 10 Hydroxylgruppen.

8. Verfahren nach einem der vorhergehenden Ansprüche, wobei dem Reaktionsmedium ein vollständig acryliertes Polyolacrylat hinzugefügt wird, wobei das Polyolacrylat der Ester einer Acrylsäureverbindung und eines Polyols gemäß der Definition in einem der vorhergehenden Ansprüche ist.

9. Verfahren nach einem der vorhergehenden Ansprüche, wobei das Polyol ausgewählt ist unter Glycerin, Diglcyerin, Triglycerin, 1,2,4- Butantriol, Trimethylolmethan, Trimethylolethan, Trimethylolpropan, Trimethylolbutan, 2,2,4-Trimethyl-1,3-pentandiol, Pentaerythrit, Ditrimethylolpropan, Dipentaerythrit, Tripentaerythrit, D-, L- und Mesoerythrit, D- und L- Arabit, Adonit, Xylit, Sorbit, Mannit, Dulcit und Inositole sowie der Mischungen und Alkoxylate, vorzugsweise Ethoxy- bzw. Propoxylate davon.

10. Verfahren nach einem der vorherigen Ansprüche, wobei das organische Lösungsmittel ausgewählt ist unter $C_1$-$C_6$-Alkanolen, Pyridin, Polyalkylenglykoldialkylether, Alkylencarbonat, $C_1$-$C_8$-Alkyl-alkancarbonsäureester, Aceton, 1,4-Dioxan, 1,3-Dioxolan, THF, Dimethoxymethan, Dimethoxyethan, und Mischungen davon.

11. Verfahren nach einem der vorherigen Ansprüche, wobei der Enzymgehalt im Reaktionsmedium im Bereich von etwa 0,01 bis 10 Gew.-%, bezogen auf das eingesetzte Polyol liegt.

12. Verfahren nach einem der vorherigen Ansprüche, wobei die Reaktionstemperatur im Bereich von 0 bis etwa 100°C liegt.

13. Verfahren nach einem der vorherigen Ansprüche, wobei das Reaktionsmedium ein- oder mehrphasig ist und worin die Reaktanden gelöst, suspendiert oder emulgiert vorliegen.

14. Verfahren nach einem der vorherigen Ansprüche, wobei man während der Umesterung anfallenden Alkohol oder bei der Veresterung anfallendes Reaktionswasser aus dem Reaktionsgleichgewicht entfernt.

15. Verfahren zur Herstellung von polymeren Polyolacrylaten, wobei man wenigstens ein Polyolacrylat nach einem Verfahren gemäß einem der vorherigen Ansprüche herstellt; das Polyolacrylat aus dem Reaktionsgemisch gegebenenfalls abtrennt; und, gegebenenfalls zusammen mit weiteren Comonomeren, polymerisiert.

16. Verfahren nach Anspruch 15, wobei man ein Polyolacrylat, das im Wesentlichen Polyol-mono-acrylate enthält, mit wenigstens einem Comonomeren zu einem linearen Copolymerisat umsetzt.

17. Verfahren nach einem der Ansprüche 1 bis 14, wobei man ein Polyolacrylatenthaltendes Reaktionsprodukt enthält mit einem molaren Anteil an Verbindungen, die sowohl Alkohol- als auch Acrylat-Funktionalisierung aufweisen, von etwa 60 bis 100 Mol-%, bezogen auf die Gesamtmolzahl Polyolacrylat.

18. Verfahren zur Herstellung von Lacken, umfassend die Herstellung eines polymeren Polyolacrylats nach einem Verfahren gemäß einem der Ansprüche 15 und 16.

19. Verfahren nach Anspruch 18 zur Herstellung von strahlungshärtbaren und / oder thermisch härtbaren Lacken.

20. Verfahren nach Anspruch 18, wobei die Lacke einen gesamtextrahierbaren Anteil von weniger als 20 Gew.-% aufweisen.

21. Verfahren nach Anspruch 20, wobei der Lack thermisch gehärtet ist.


**Claims**

1. A process for the enzymatic synthesis of polyol acrylates, in which an aliphatic polyol is reacted with an acrylic acid compound or an alkyl ester thereof in bulk or in a liquid reaction medium comprising an organic solvent, in the presence of an enzyme which is selected from lipases (E.C.3.1.1.3) and transfers acrylate groups, and after the end of the reaction the polyol acrylate(s) formed is (are) isolated optionally from the reaction mixture.

**2.** The process according to claim 1, wherein a lipase from *Candida sp.,* more particularly *Candida antarctica* B, or *Burkholderia sp.* is used.

**3.** The process according to claim 1 or 2, wherein the liquid reaction medium has an initial water content of less than about 10% by volume.

**4.** The process according to either of the preceding claims, wherein acrylic acid compound and polyol are used in a molar ratio of about 100:1 to 1:1.

**5.** The process according to any of the preceding claims, wherein the acrylic acid compound is selected from acrylic acid, lower-alkylsubstituted acrylic acid, and the alkyl esters of these compounds, and also mixtures thereof.

**6.** The process according to any of the preceding claims, wherein the polyol is selected from straight-chain or branched or carbocyclic, saturated or unsaturated hydrocarbon compounds having at least 3 carbon atoms and at least 3 (esterifiable) hydroxyl groups in optically pure form or as a stereoisomer mixture, or mixtures of different polyols.

**7.** The process according to claim 6, wherein the polyol is selected from straight-chain, branched or cyclic saturated hydrocarbons having 3 to 30 carbon atoms and from 3 to 10 hydroxyl groups.

**8.** The process according to any of the preceding claims, wherein a completely acrylated polyol acrylate is added to the reaction medium, the polyol acrylate being the ester of an acrylic acid compound and a polyol as defined in any of the preceding claims.

**9.** The process according to any of the preceding claims, wherein the polyol is selected from glycerol, diglycerol, triglycerol, 1,2,4-butanetriol, trimethylolmethane, trimethylolethane, trimethylolpropane, trimethylolbutane, 2,2,4-trimethyl-1,3-pentanediol, pentaerythritol, ditrimethylolpropane, dipentaerythritol, tripentaerythritol, D-, L-, and mesoerythritol, D- and L-arabitol, adonitol, xylitol, sorbitol, mannitol, dulcitol and inositols, and also the mixtures and alkoxylates, preferably ethoxylates and/or propoxylates, thereof.

**10.** The process according to any of the previous claims, wherein the organic solvent is selected from $C_1$-$C_6$ alkanols, pyridine, polyalkylene glycol dialkyl ethers, alkylene carbonate, $C_1$-$C_6$ alkyl alkanecarboxylic esters, acetone, 1,4-dioxane, 1,3-dioxolane, THF, dimethoxymethane, dimethoxyethane, and mixtures thereof.

**11.** The process according to any of the previous claims, wherein the enzyme content of the reaction medium is in the range from about 0.01 to 10% by weight, based on the polyol used.

**12.** The process according to any of the previous claims, wherein the reaction temperature is in the range from 0 to about 100°C.

**13.** The process according to any of the previous claims, wherein the reaction medium is single-phase or multiphase and wherein the reactants are present in solution, suspension or emulsion.

**14.** The process according to any of the previous claims, wherein alcohol produced during the transesterification or water of reaction produced during the esterification is removed from the reaction equilibrium.

**15.** A process for preparing polymeric polyol acrylates, wherein at least one polyol acrylate is prepared by a process according to any of the previous claims, separated if desired from the reaction mixture, and polymerized if desired together with further comonomers.

**16.** The process according to claim 15, wherein a polyol acrylate comprising substantially polyol monoacrylates is reacted with at least one comonomer to form a linear copolymer.

**17.** The process according to any of claims 1 to 14, wherein a reaction product comprising polyol acrylate is obtained with a molar fraction of compounds having both alcohol functionalization and acrylate functionalization of about 60 to 100 mol%, based on the total molar number of polyol acrylate.

**18.** A process for the preparation of coating materials, including the preparation of a polymeric polyol acrylate by a process according to either of claims 15 and 16.

**19.** The process according to claim 18 for the preparation of radiation-curable and/or thermally curable coating materials.

**20.** The process according to claim 18, wherein the coating materials have a total extractable fraction of less than 20% by weight.

**21.** The process according to claim 20, wherein the coating material is thermally cured.

**Revendications**

**1.** Procédé pour la synthèse enzymatique d'acrylates de polyol, un polyol aliphatique étant transformé en présence d'une enzyme transférant des groupes acrylate choisie parmi les lipases (E.C.3.1.1.3), en masse ou dans un milieu réactionnel liquide, comprenant un solvant organique, avec un composé d'acide acrylique ou un ester d'alkyle de celui-ci et le(s) acrylate(s) de polyol formé(s) étant le cas échéant isolé(s) du mélange réactionnel après la fin de la réaction.

**2.** Procédé selon la revendication 1, une lipase parmi Candida sp., en particulier Candida antarctica B, ou Burkholderia sp, étant utilisée.

**3.** Procédé selon la revendication 1 ou 2, le mélange réactionnel liquide présentant une teneur en eau de départ inférieure à 10% en volume.

**4.** Procédé selon l'une quelconque des revendications précédentes, le composé d'acide acrylique et le polyol étant utilisés dans un rapport molaire d'environ 100:1 à 1:1.

**5.** Procédé selon l'une quelconque des revendications précédentes, le composé d'acide acrylique étant choisi parmi l'acide acrylique, l'acide acrylique substitué par $C_1$-$C_6$-alkyle et les esters d'alkyle de ces composés ainsi que leurs mélanges.

**6.** Procédé selon l'une quelconque des revendications précédentes, le polyol étant choisi parmi les composés hydrocarbonés linéaires ou ramifiés ou carbocycliques, saturés ou insaturés, comprenant au moins 3 atomes de carbone et au moins 3 groupes hydroxyle (estérifiables) sous une forme optiquement pure ou sous forme d'un mélange de stéréo-isomères ou de mélanges de différents polyols.

**7.** Procédé selon la revendication 6, le polyol étant choisi parmi les hydrocarbures linéaires, ramifiés ou cycliques, saturés, comprenant 3 à 30 atomes de carbone et 3 à 10 groupes hydroxyle.

**8.** Procédé selon l'une quelconque des revendications précédentes, un acrylate de polyol complètement acrylé étant ajouté au milieu réactionnel, l'acrylate de polyol étant l'ester d'un composé d'acide acrylique et d'un polyol selon la définition dans l'une quelconque des revendications précédentes.

**9.** Procédé selon l'une quelconque des revendications précédentes, le polyol étant choisi parmi le glycérol, le diglycérol, le triglycérol, le 1,2,4-butanetriol, le triméthylolméthane, le triméthyloléthane, le triméthylolpropane, le triméthylolbutane, le 2,2,4-triméthyl-1,3-pentanediol, le pentaérythritol, le ditriméthylolpropane, le dipentaérythritol, le tripentaérythritol, le D-érythritol, le L-érythritol et le méso-érythritol, le D-arabitol et le L-arabitol, l'adonitol, le xylitol, le sorbitol, le mannitol, le dulcitol et les inositols ainsi que leurs mélanges et leurs alcoxylates, de préférence leurs éthoxylates ou, selon le cas, propoxylates.

**10.** Procédé selon l'une quelconque des revendications précédentes, le solvant organique étant choisi parmi les $C_1$-$C_6$-alcanols, la pyridine, les polyalkylèneglycoldialkyléthers, les alkylènecarbonates, les esters d'acide $C_1$-$C_6$-alkylalcanecarboxylique, l'acétone, le 1,4-dioxane, le 1,3-dioxolane, le THF, le diméthoxyméthane, le diméthoxyéthane et leurs mélanges.

**11.** Procédé selon l'une quelconque des revendications précédentes, la teneur en enzyme dans le milieu réactionnel se situant dans la plage d'environ 0,01 à 10% en poids, par rapport au polyol utilisé.

**12.** Procédé selon l'une quelconque des revendications précédentes, dans lequel la température de réaction se situe dans la plage de 0 à environ 100°C.

**13.** Procédé selon l'une quelconque des revendications précédentes, le milieu réactionnel étant à une ou plusieurs phases et dans lequel les réactifs se trouvent sous forme dissoute, en suspension ou en émulsion.

**14.** Procédé selon l'une quelconque des revendications précédentes, l'alcool produit pendant la transestérification ou l'eau de réaction produite pendant la réaction étant éliminé(e) de l'équilibre de réaction.

**15.** Procédé pour la préparation d'acrylates de polyol polymères, au moins un acrylate de polyol étant préparé selon un procédé selon l'une quelconque des revendications précédentes ; l'acrylate de polyol étant le cas échéant séparé du mélange réactionnel ; et polymérisé, le cas échéant ensemble avec d'autres comonomères.

**16.** Procédé selon la revendication 15, un acrylate de polyol, qui contient principalement des monoacrylates de polyol, étant transformé avec au moins un comonomère en un copolymère linéaire.

**17.** Procédé selon l'une quelconque des revendications 1 à 14, un produit de réaction contenant un acrylate de polyol étant obtenu, présentant une proportion molaire de composés qui présentent tant une fonctionnalisation alcool qu'une fonctionnalisation acrylate, d'environ 60 à 100% en mole, par rapport au nombre total de moles d'acrylate de polyol.

**18.** Procédé pour la préparation de laques comprenant la préparation d'un acrylate de polyol polymère selon l'une quelconque des revendications 15 et 16.

**19.** Procédé selon la revendication 18 pour la préparation de laques durcissables par des rayons et/ou thermodurcissables.

**20.** Procédé selon la revendication 18, les laques présentant une proportion totale pouvant être extraite de moins de 20% en poids.

**21.** Procédé selon la revendication 20, la laque étant thermodurcie.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 59220196 A
- US 5240835 A
- EP 999229 A
- DE 10156352 A
- DE 19856948 A1
- EP 0999229 A
- US 6359101 B
- DE 19817676
- DE 19913260
- US 6429342 B
- US 6077979 A
- US 5545601 A
- WO 9800456 A
- EP 92269 A
- EP 7508 A
- EP 57474 A
- DE 19618720 A
- EP 495751 A
- EP 615980 A
- DE 19826712 A
- DE 19913353 A
- WO 9833761 A
- DE 19957900 A1

**In der Beschreibung aufgeführte Nicht-Patentliteratur**

- **DERANGO et al.** *Biotechnol Lett.,* 1994, vol. 16, 241-246
- **ATHAWALE ; MANJREKAR.** *J. Mol. Cat. B Enzym.,* 2000, vol. 10, 551-554
- **POTIER et al.** *Tetrahedron Lett.,* 2000, vol. 41, 3597-3600
- **WARWEL et al.** *Biotechnol Lett.,* 1996, vol. 10, 283-286
- **NUROK et al.** *J. Mol. Cat. B Enzym.,* 1999, vol. 7, 273-282
- **HAJJAR et al.** *Biotechnol. Lett.,* 1990, vol. 12, 825-830
- **TOR et al.** *Enzym. Microb. Technol.,* 1990, vol. 12, 299-304
- **WARWEL S. et al.** An efficient method for lipase-catalysed preparation of acrylic and methacrylic acid esters. *BiOTECHNOLOGY TECHNIQUES,* 01. April 1996, vol. 10 (4), 283-286
- Bioreaktor. Römpp Chemie Lexikon. Thieme Verlag
- Ullmann's Encyclopedia of Industrial Chemistry. vol. B4, 381 ff
- Ullmann's Enzyclopedia of Industrial Chemistry. 2000
- Advances in Polymer Science. Springer, 1974, vol. 14
- **K. K. DIETLIKER.** *Chemistry and Technology of UV- and EB-Formulation for Coatings, Inks and Paints,* vol. 3
- Photoinitiators for Free Radical and Cationic Polymerization. SITA Technology Ltd
- Polymer Handbook. Wiley & Sons